# EUROPEAN PATENT SPECIFICATION

(11) **EP 4 211 648 B1**
(45) Date of publication and mention of the grant of the patent: **22.07.2026**
(21) Application number: 21778226.7
(22) Date of filing: 07.09.2021
(51) Int. Cl.: A61B 6/00, A61B 6/50, A61B 6/04, A61B 6/02, G06T 7/00, G06T 7/30

(54) **METHOD FOR AIDING AN OPERATOR TO DETERMINE A POSITION OF A REGION OF INTEREST IN A BREAST AND MAMMOGRAPHY APPARATUS IMPLEMENTING IT**
VERFAHREN ZUR UNTERSTÜTZUNG EINES BEDIENERS ZUR BESTIMMUNG EINER POSITION EINER REGION VON INTERESSE IN DER BRUST UND MAMMOGRAPHIEVORRICHTUNG DAMIT
MÉTHODE POUR AIDER UN OPÉRATEUR À DÉTERMINER UNE POSITION D'UNE RÉGION D'INTÉRÊT DANS UN SEIN ET APPAREIL DE MAMMOGRAPHIE LA METTANT EN OEUVRE

(30) Priority: 09.09.2020 IT 202000021274
(43) Date of publication of application: 19.07.2023
(73) Proprietor: IMS Giotto S.p.A., 40037 Sasso Marconi (Bologna) (IT)
(72) Inventor: VECCHIO, Sara, 40033 Casalecchio di Reno (BO) (IT); MUSCA, Lorenzo, 40033 Casalecchio di Reno (BO) (IT); VIGNOLI, Paolo, 40017 San Giovanni in Persiceto (BO) (IT)
(74) Representative: Bird & Bird Società tra Avvocati S.r.l.
(86) International application number: PCT/IB2021/058115
(87) International publication number: WO 2022/053928

(56) References cited:
- EP-A1- 3 320 844
- US-A1- 2016 007 943
- US-A1- 2018 333 109
- JAN EHRHARDT ET AL: "Automatic correspondence detection in mammogram and breast tomosynthesis images", PROCEEDINGS OF SPIE, vol. 8314, 10 February 2012 (2012-02-10), pages 831421, XP055207114, ISSN: 0277-786X, DOI: 10.1117/12.911305

## Description

### FIELD OF THE INVENTION

The present invention relates to a method for aiding an operator to determine a position of a region of interest in a breast and a mammography apparatus implementing it.

The region of interest corresponds in particular to a portion of the breast from which a sample is to be withdrawn for diagnostic purposes, such as biopsy procedures.

### BACKGROUND ART

It is known to guide breast biopsy procedures using 3D breast imaging systems. Such a solution is disclosed and shown, for example, in Patent Document EP3023076A1.

More recently, solutions combining both 3D breast imaging procedures of the "tomosynthesis" type and 2D breast imaging procedures have been proposed. Solutions of this kind are disclosed and shown, for example, in Patent Documents EP2491863A1 and EP2656789A1.

### SUMMARY

The general object of the present invention is to provide an alternative and improved solution with respect to the known ones.

This object is achieved by the method and apparatus having the characteristics explained in the herein appended claims which form an integral part of the present invention.

According to the present invention, both a 3D breast imaging procedure of the "tomosynthesis" type and a 2D breast imaging procedure of the "dual energy" or "DEM" type, in particular and preferably of the "CESM" (= Contrast-Enhanced Spectral Mammography) type, are combined; indeed, advantageously, "dual-energy" mammography, in particular that carried out with a contrast medium, allows particularly effective visualisation for performing biopsies.

"Dual energy mammography" or "DEM" is a well-known technique whereby two images of a breast are taken from the same position in rapid succession (e.g., seconds or less). The two images are obtained by irradiating the breast with two different spectra (in the X-ray range) because they are centred on two different average energies. There is also an alternative known technique whereby the breast is irradiated using the same spectrum and the two images are obtained by performing a spectral separation using a detector capable of discriminating the energy of the photons striking it. Hence, in both cases, each of the two images is associated with a different spectrum (and its relative average energy).

According to preferred embodiments of the present invention, 2D imaging occurs prior to 3D imaging; it is thereby possible, for example, to centre the breast according to DEM or CESM before proceeding with tomosynthesis and subsequently biopsy. If the DEM or CESM possibly indicates that the breast is not well positioned (in particular, if it is established that the biopsy needle cannot reach a certain lesion on the breast, for example because that lesion is not displayed within an appropriate for accessing to/withdrawing from the breast), it can be repositioned by relieving or removing the pressure on the breast by the mammography apparatus "compression plate" or "compressor". Experts in the field use the term "scout image" to identify an image which is primarily used for visually searching for lesions.

In order to benefit from the advantages of both types of imaging, the present invention proposes to enable markers to be displayed on the 2D image and/or on one or more "sections" of the 3D image, and to ensure a correspondence between the 2D image and the "sections" of the 3D image as well as between the markers on the two types of images.

This is achieved by three-dimensional to two-dimensional mapping and two-dimensional transformation operations that pass through a virtual 2D image created from the 3D image.

The problem of matching a mammographic image with a tomosynthesis image is addressed, for example, in the article by Jan Ehrhardt et al. entitled "Automatic Correspondence Detection in Mammogram and Breast Tomosynthesis Images" (Proceeding of SPIE, vol. 8314, 2012). The article proposes two approaches.

According to the first approach, by means of an iterative process, the tomosynthetic image, which is three-dimensional, is registered against the mammographic image, which is two-dimensional, through a repeated generation of "DRR" (which are two-dimensional images that can be considered as "virtual") and repeated optimisation of the similarity between "Mammogram" and "DRR". According to the second approach, the mammographic image and the central projection of the tomosynthetic image are firstly registered with each other and then each of the other projections of the tomosynthetic image is registered with respect to the central one; these registrations are not based on "virtual" images.

These approaches are not suitable for use in guiding a biopsy procedure. In fact, both of them modify the tomosynthetic image that is at the very basis of a guided biopsy procedure. In addition, both of them require heavy and therefore time-consuming calculations. Therefore, these approaches can only be used for displaying purposes, and, moreover, are designed for images obtained at very different times (at least hours, but typically days or weeks or months) and therefore not during the same compression operation.

An advantage of the approach according to the present invention over a possible stereotactic examination performed entirely in a "dual energy" mode is that examination times are shortened. Experts in the field know that the tomosynthesis image-guided biopsy method significantly shortens the procedure times. This approach is therefore extremely advantageous for the comfort of the patient (who is subjected to discomfort and anxiety during breast imaging procedures and possible subsequent biopsy), but even more important in the case of performing imaging procedures with concurrent injection of a contrast medium, which has a limited residence time inside the human body.

An advantage of the approach according to the present invention over a possible tomo-guided biopsy performed entirely in a "dual energy" mode is the significant reduction in the X-ray dose absorbed by the patient being examined; in fact, the X-ray delivery for a "dual energy" tomosynthesis corresponds to twice the delivery for a "single energy" (i.e., conventional) tomosynthesis. In addition, the CEST (= Contrast-Enhanced Spectral Tomosynthesis) technique, or "dual energy" tomosynthesis, involves complications as artefacts typical of CESM and artefacts typical of tomosynthesis combine together, resulting in problems with the visibility of the lesions.

In order to amplify the correlation between the two types of imaging, the energy of 3D imaging can be chosen appropriately and advantageously; this choice can be made according to optimisation criteria that differ from the general criterion of anatomical visibility in conventional mammography; for example, choice criteria can be used that are similar to those for optimising the acquisition with energy further away from the mammographic range in dual energy imaging. If, for example, during a biopsy procedure, the scout image derives from a CESM, it must be assumed that an attempt is being made to take a tissue sample from a contrast-medium highly capturing area. In this case, the tomosynthesis image will therefore preferably be taken at an energy such that there is a photon absorption peak by the contrast medium, neglecting the optimisation of the visibility of the surrounding tissues. This energy is presumably equal to or nearly equal to the highest energy of the pair of energies used for CESM.

A further advantage of the imaging combination according to the present invention is given by the possibility of inserting an anti-scatter grid during scout image acquisition. In normal stereotactic examinations, the standard method of removing scattered radiation, i.e., using an anti-scatter grid, cannot be used because the stereotactic projections are not compatible with the vertical focusing direction of the grid. In order to make the display of the stereotactic images and the (vertical) image used for centring homogeneous, the anti-scatter grid is not used for the "scout" image either. However, this results in a different (degraded) image quality if compared to the mammographic images on the basis of which the patient was probably recommended for further diagnosis with the biopsy. As, according to the present invention, the "scout" image acquisition mode (DEM or CESM) and the image acquisition mode used for pointing (tomosynthesis) are already naturally non-homogeneous, the anti-scatter grid can be advantageously used in the "scout" image acquisition (DEM or CESM).

According to the present invention, it is advantageously possible to perform a strong relation between two images of different types; registration techniques are employed which involve, on the one hand, a composite 2D image deriving from a CESM or one of the two images underlying a CESM and, on the other hand, a virtual 2D image obtained by mapping on a plane a 3D image deriving from a tomosynthesis, and which are advantageous especially if the two different images are acquired in sequence and if they are spaced by a relatively long time distance (e.g. of the order of one minute) during the same compression operation; in fact, in this case, there are discrepancies (more or less evident) in the observed anatomy due to relatively small movements of the patient, e.g. of the order of a millimetre.

### LIST OF FIGURES

The present invention shall become more readily apparent from the detailed description that follows to be considered together with the accompanying drawings in which:
Fig. 1 shows a block diagram of an embodiment of an apparatus according to the present invention,
Fig. 2 schematically shows the performance of a 3D tomosynthesis-type imaging procedure with the apparatus of Fig. 1,
Fig. 3 schematically shows how a virtual 2D image is generated by mapping a 3D tomosynthesis-type image with the apparatus of Fig. 1,
Fig. 4 shows a flow chart of an embodiment of the method according to the present invention,
Fig. 5 shows an example of a "low energy" image acquired during a 2D breast CESM imaging procedure,
Fig. 6 shows an example of a "high energy" image acquired during a 2D breast CESM imaging procedure,
Fig. 7 shows a "composite" image corresponding to a combination of the images in
Fig. 5 and Fig. 6,

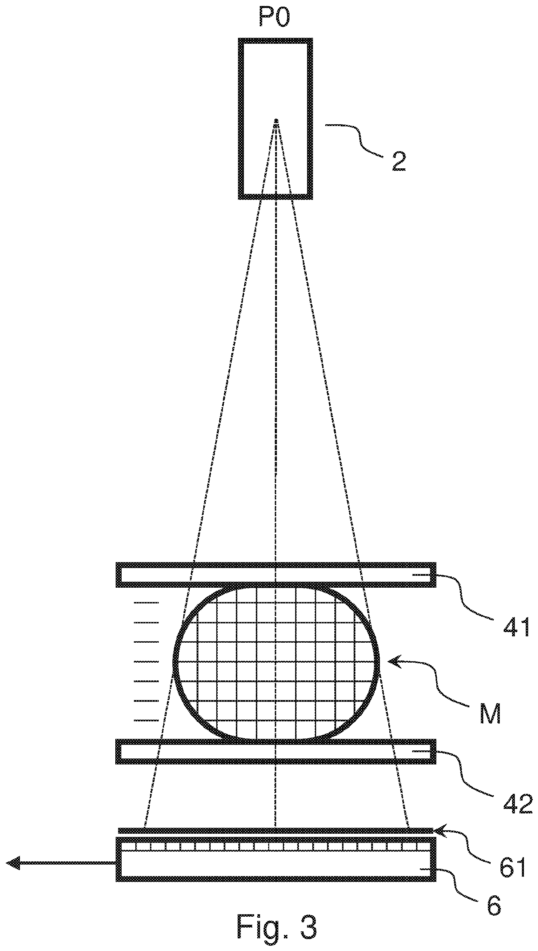

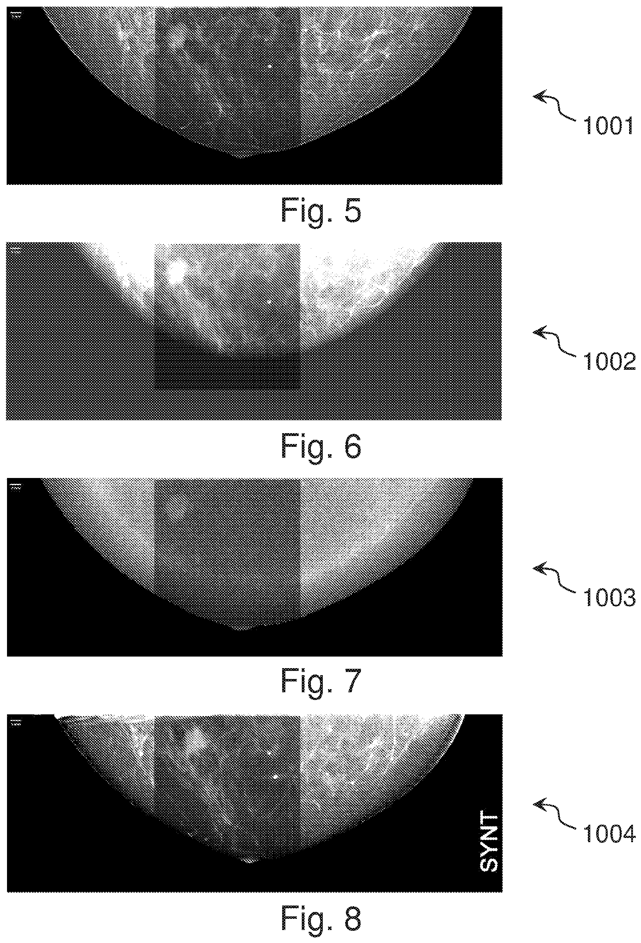

Fig. 8 shows an example of a "virtual" breast image obtained by mapping a 3D tomosynthesis-type image as shown in Fig. 3, and

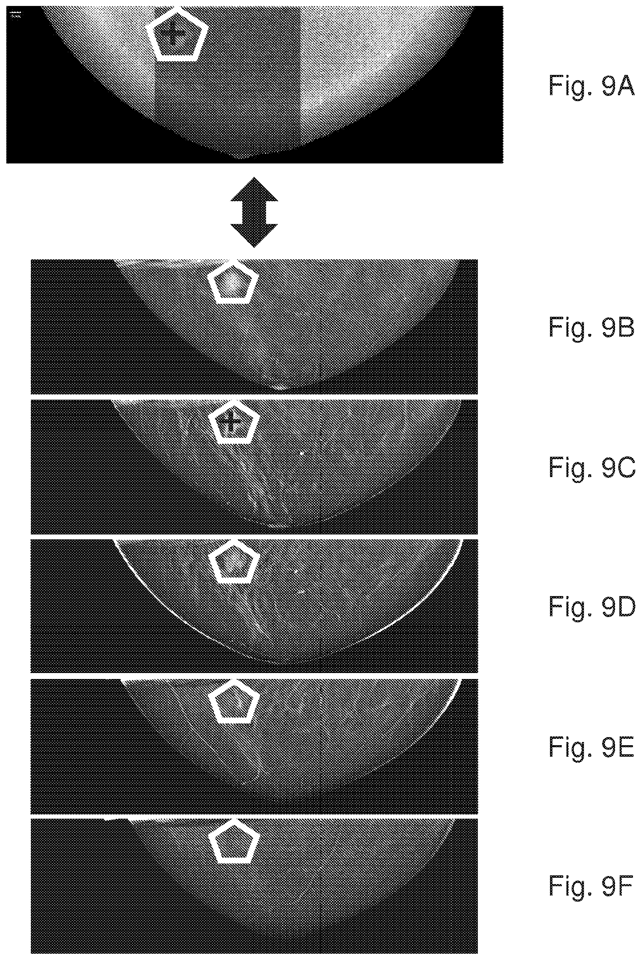

Fig. 9 shows the display, according to the present invention, of corresponding marking elements in various images.

As it can be easily understood, there are various ways of practically implementing the present invention which is defined in its main advantageous aspects in the appended claims and is not limited either to the following detailed description or to the appended claims.

### DETAILED DESCRIPTION

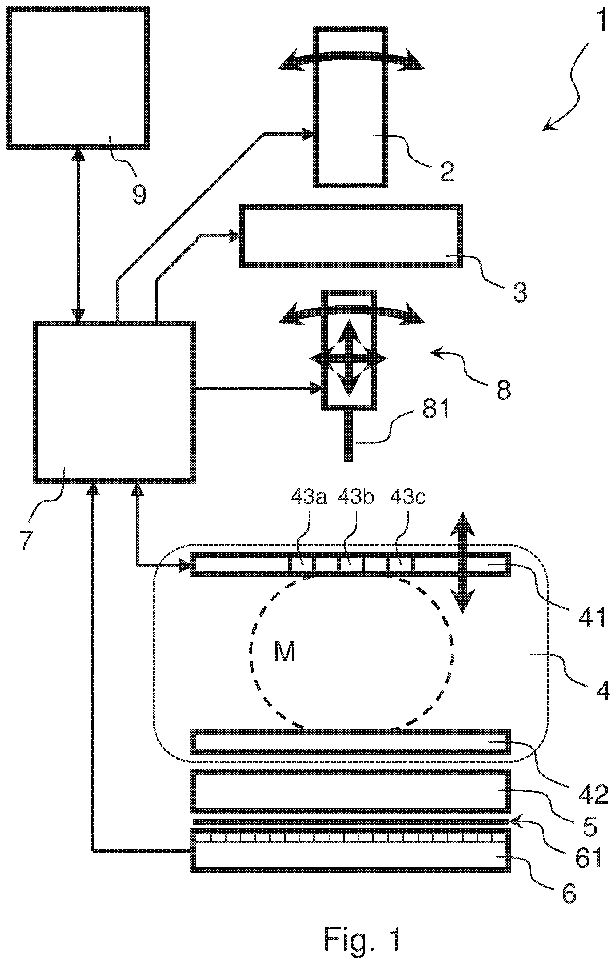

The apparatus 1 of Fig. 1 comprises an X-ray emitter 2 and an X-ray detector 6 adapted to detect the X-rays emitted by emitter 2.

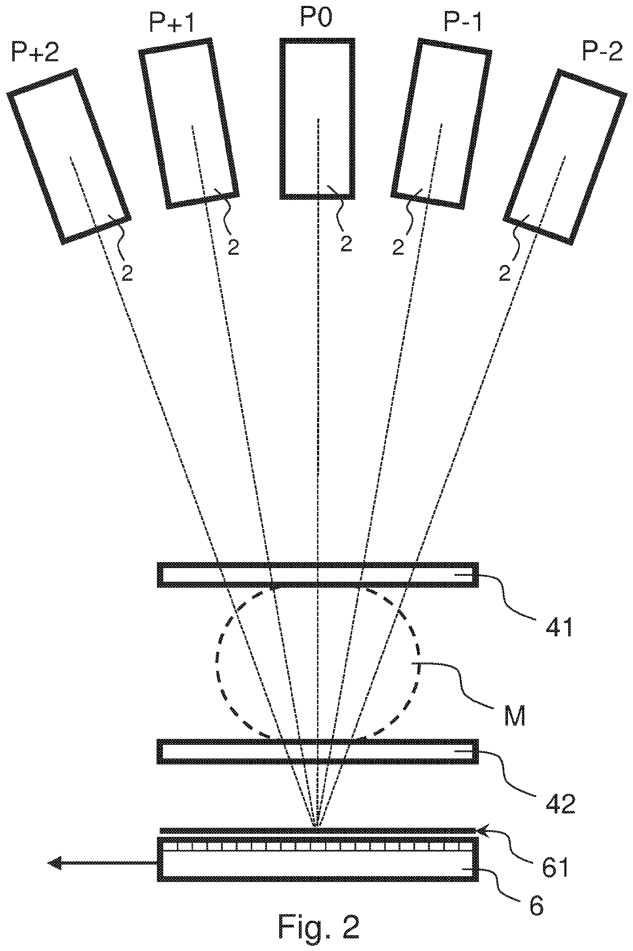

As it is clear, Fig. 1, Fig. 2 and Fig. 3 correspond to each other and (partially) show the same components as apparatus 1. Fig. 2 and Fig. 3 focus on certain aspects of the apparatus 1 and therefore some components (in particular the filter assembly 3 and the grid assembly 5) are not shown, but only for the sake of clarity.

In particular and as graphically highlighted, the detector 6 is of electronic type and integrates a two-dimensional array, or matrix, of elementary sensors, for example one per pixel, which defines an acquisition plane 61; in Fig. 1, Fig. 2 and Fig. 3, the plane 61 is shown as if it was external to the detector 6, on the contrary it is internal and may correspond to the top surface of the detector or to a parallel plane at a small distance (e.g. 0.3-3.0 mm). It should be noted that alternative electronic sensors integrating a one-dimensional array of elementary sensors, e.g., one per pixel, adapted to perform a translational image scanning motion, are known in the literature.

A first function of the apparatus 1 is therefore to perform breast imaging procedures; in Fig. 1, Fig. 2 and Fig. 3, a breast is schematised with an oval M in a position such to intercept the X-rays emitted by the emitter 2 before they hit the detector 6; obviously, part of the X-rays emitted by the emitter 2 are not intercepted by the breast M.

Fig. 1 shows that the emitter 2 may be moved, in particular rotated about an axis that lies in the acquisition plane 61 and is perpendicular to the plane of Fig. 1; this rotation may be better understood by looking at Fig. 2 and is used to perform 3D tomosynthesis-type imaging procedures. However, in general, other possible movements of the emitter 2, in particular translation and/or rotation, should not be excluded.

A second function of the apparatus 1 is therefore to perform breast biopsy procedures. In Fig. 1 a biopsy assembly 8 equipped with a suitable biopsy needle 81 and its withdrawal mechanism for inserting and withdrawing the needle to take the sample is schematically shown. In general, the assembly 8 can move in different ways, in particular translating (e.g., in one, two or three directions) and rotating (e.g., around one, two or three axes), in order to best fulfil its function.

The apparatus 1 further comprises a breast positioning assembly 4 conceptually consisting of an upper contact element 41 and a lower contact element 42 between which a breast M is compressed. In particular, the element 42 is fixed with respect to the detector 6 and the element 41 is movable with respect to the element 42 in that it can firstly move away to insert the breast M and then move closer by compressing the breast M. The element 41, called "compression plate" and hereinafter "compressor" for ease of brevity, may be adapted to receive electrical position control signals and/or transmit electrical measurement signals, such as force and/or position. In Fig. 1, the compressor 41 is equipped with several windows to allow the passage of a biopsy needle, in particular the needle 81; the figure shows for example three windows 43a, 43b and 43c, but the number could be different; moreover, it cannot be excluded that, thanks to the range of possible movements of the assembly 8, the needle 81 could penetrate the breast M from a side rather than from above.

In Fig. 1, two other possible components are shown, a filter assembly 3 and a grid assembly 5. The former, in combination with the emitter 2, is used to shape the spectrum of X-rays entering the breast; in particular, to perform DEM or CESM procedures, the "low energy"/"high energy" effect can be obtained by choosing the high (anodic) voltage to be supplied as input to the emitter 2 and the assembly 3 filter to be used for filtering. The second one is for improving the quality of the image detected by detector 6 as it reduces the contribution of scattering, which results in a general blurring of details.

No further details of the components mentioned above are provided herein because they are in themselves known in the field.

Two important components of the apparatus 1 shown in Fig. 1 need to be highlighted for the purposes of the present invention: a processing and control unit 7 and a human-machine interface unit 9.

Unit 7 is electrically connected to all the other components and is the one that basically determines the operation of apparatus 1. It is, in general, a computerised unit equipped with a system software and possibly an application.

Unit 9 is connected to unit 7 and allows the operator to provide "inputs", e.g., data and/or commands, to the apparatus 1 in particular unit 7, and to receive "outputs" from the apparatus 1 in particular from unit 7. Typically, the unit 9 is a computerised unit equipped with an application software, in particular a PC, and comprises at least one screen, a keyboard and a mouse and/or joystick, as well as other devices for controlling, for example, the movements of the various components of the apparatus and the delivery of X-rays.

Typically, thanks to the appropriate software of the processing and control unit and/or thanks to the human-machine interface unit with relative appropriate software, it is possible to carry out the methods according to the present invention. The components of unit 7 and unit 9 are not shown in Fig. 1 for ease of simplicity. It is worth bearing in mind that Fig. 1 is highly schematic and therefore no deductions should be made about the position and composition of the processing and control unit.

Fig. 2 schematises the performance of a 3D tomosynthesis-type imaging procedure by means of the apparatus of Fig. 1 on the breast M (taking into account that in Fig. 2 the assembly 3 is not shown but is present as in Fig. 1); this figure assumes, by way of example, that the resulting 3D image is derived from five consecutive detections by the detector 6.

While the breast M is compressed between elements 41 and 42:
- the emitter 2 is positioned at position P-2 and delivers X-rays, for example, pointing to a central point of the detector 6, detector 6 detects a 2D image on the acquisition plane 61 and sends it to the unit 7, then
- the emitter 2 is positioned at position P-1 and delivers X-rays, for example, pointing to the central point of the detector 6, the detector 6 detects a 2D image on the acquisition plane 61 and sends it to the unit 7, then
- the emitter 2 is positioned at position P0 and delivers X-rays, for example, pointing to the central point of the detector 6, the detector 6 detects a 2D image on the acquisition plane 61 and sends it to the unit 7, then
- the emitter 2 is positioned at position P+1 and delivers X-rays, for example, pointing to the central point of the detector 6, the detector 6 detects a 2D image on the acquisition plane 61 and sends it to the unit 7, then
- the emitter 2 is positioned at position P+2 and delivers X-rays, for example pointing to the central point of the detector 6, the detector 6 detects a 2D image on the acquisition plane 61 and sends it to the unit 7, finally
- the unit 7 processes all the 2D images received and generates a 3D tomosynthesis-type image consisting of a three-dimensional matrix of voxels;
the positions P-2, P-1, P0, P+1 and P+2 correspond to the positions of the "focal point" (it is more precisely a "focal spot") of the emitter 2 and are ideally located on a circumference centred for example on the central point of the detector 6 in the acquisition plane 61, which we could call "acquisition centre"; the axis joining the "focal spot" and the "acquisition centre" is rotated, for the various positions, for example by -20°, -10°, 0°, +10° and +20° respectively.

Delivering X-rays on various occasions for acquiring the above 3D image is performed using a certain voltage (anodic), which is always the same, and with a certain filtering, which is always the same.

Rather than the point of the detector 6 in the acquisition plane 61, another point can be used that can be located for example on a central axis perpendicular to the detector 6 preferably between the acquisition plane 61 and the plane associated with the element 41.

Performing a 2D DEM or CESM imaging procedure with the apparatus of Fig. 1 on the breast M may still be described with the aid of Fig. 2, bearing in mind that in this figure the assemblies 3 and 5 are not shown but are present (as in Fig. 1). While the breast M is compressed between elements 41 and 42:
- the emitter 2 is positioned in position P0, the assembly 3 is set for a first type of filtering, the emitter 2 performs a first delivery of X-rays using a first voltage (anodic) and pointing for example (perpendicularly) to the central point of the detector 6, the detector 6 detects a first 2D image on the acquisition plane 61 which is for example filtered through the assembly 5 and sends it to unit 7 - it can be for example a "low energy" image (for example 20 KeV), and then
- the emitter 2 remains in position P0, the assembly 3 is set for a second type of filtering, the emitter 2 makes a second X-ray delivery using a second (anodic) voltage and pointing for example (perpendicularly) to the central point of the detector 6, the detector 6 detects a second 2D image on the acquisition plane 61 which is for example filtered through the assembly 5 and sends it to the unit 7 - this may be for example a "high energy" image (e.g. 36 KeV).

Typically, the unit 7 processes the two received 2D images and generates a composite 2D image comprising a 2D matrix of pixels corresponding to a combination of the first image and the second image.

Fig. 3 will be considered hereinafter in detail, at the moment it is sufficient to note that in this figure the emitter 2 is in the same position P0 as used for the 2D DEM or CESM imaging procedure, that the breast M is compressed between elements 41 and 42 as for the 2D DEM or CESM imaging procedure, that the detector 6 (and its acquisition plane 61) is in the same position P0 as used for the 2D DEM or CESM imaging procedure. The breast M is subdivided into voxels as from a 3D image resulting from a previous tomosynthesis 3D imaging procedure: in particular, the various "sections" of the 3D image are highlighted - all horizontally aligned voxels placed at the same distance from the acquisition plane 61 belong to a "section"; they are therefore, slices with a small thickness (e.g. 0.5-1.0 mm) corresponding to one side of the voxel. In Fig. 3, we see the cone or pyramid of X-rays created by the emitter 2 (typically the emitter is associated with a "collimator" adapted to determine the shape and size of the cone or pyramid) which is intercepted by the breast M, conceptually subdivided into voxels, and which reaches the pixels of the detector 6.

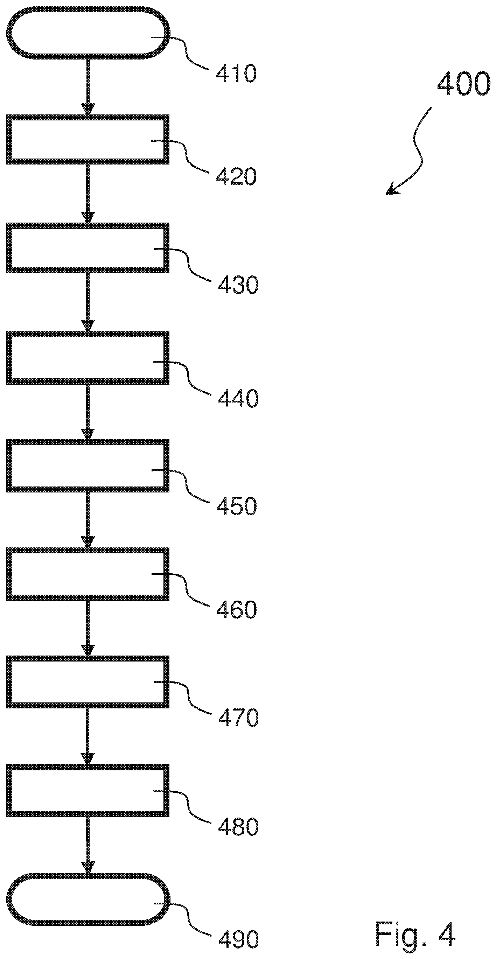

The method according to the present invention will be illustrated hereinafter with the aid of figures 1 to 3 as well as the flowchart 400 in figure 4; the references in brackets are for exemplary purposes only. Diagram 400 shows a method start block 410 and a method end block 490 with possible starting and final activities.

The method according to the present invention is for aiding an operator to determine a spatial position of a region of interest in a breast (M) in order to subsequently guide a biopsy procedure in the breast itself, for example, to firstly move appropriately the biopsy assembly (8) and then to withdraw tissue by inserting and extracting the biopsy needle (81).

In general, the method comprises the steps of:
A) performing a 2D breast imaging procedure (M); this step is subdivided into two sub-steps; a first sub-step (block 420) involves a first delivery of X-rays from a predetermined emission point (P0) at a first energy to obtain a first image (see for example image 1001 in Fig. 5), in the form of a 2D matrix of pixels, on a predetermined acquisition plane (61); a second sub-step (block 430) involves a second delivery of X-rays from the predetermined emission point (P0) at a second energy to obtain a second image (see for example image 1002 in Fig. 6), in the form of a 2D matrix of pixels, on the predetermined acquisition plane (61); the emission point (P0) and the acquisition plane (61) are to be considered as predetermined geometric parameters of the 2D imaging procedure;
B) generating a composite 2D image (see for example image 1003 in Fig. 7) (block 440) consisting of a 2D matrix of pixels corresponding to a combination of the first image (1001) and the second image (1002);
C) performing a 3D tomosynthesis-type imaging procedure (block 450) of the breast M by delivering X-rays from various emission points (P-2, P-1, P0, P+1, P+2 - see for example Fig. 2) at a third energy to obtain a 3D image consisting of a 3D matrix of voxels (see for example Fig. 3);
D) generating a virtual 2D image (see image 1004 in Fig. 8) (block 460) by means of a predetermined mapping of the 3D image onto a virtual plane corresponding to the predetermined acquisition plane (61) according to geometric mapping parameters corresponding to the predetermined geometric parameters of the 2D imaging procedure referred to in step A - this step is schematically shown in Fig. 3 and will be better explained hereinafter, but, to get an idea of step D, one can think of the virtual 2D image as corresponding, for example, to a projection of the 3D image onto the acquisition plane;
E) determining a two-dimensional transformation (block 470) linking a portion of the composite 2D image (1003) and a corresponding portion of the virtual 2D image (1004) in such a way as to register the composite 2D image (1003) with respect to the virtual 2D image (1004) and generating a registered 2D image (block 480) based on this two-dimensional transformation - this step will be better explained hereinafter, but by way of example, it can be disclosed here that its function is to take into account that external and/or internal micro-movements of the breast under examination certainly occurred between the moment when the two images of the dual energy mammography in step A were obtained and the moment when the images of the tomosynthesis in step C were obtained, - it should be noted that, alternatively, rather than using the composite 2D image of step B to determine the transformation, either the first image or the second image of the 2D imaging procedure of step A can be used; e
F) using the geometric parameters of the mapping to display (block 490) at least one marking point; this point has been previously located manually by an operator or automatically by a computer program or by an operator with the aid of a program; this will typically also be highlighted; there are in fact two possibilities: either the point is first located in a section of the 3D image and then displayed in the 2D (dual energy) registered image or the point is first located in the 2D (dual energy) registered image and then displayed in a section of the 3D image.

It should be noted that the 2D imaging procedure and the 3D imaging procedure are performed on a breast in a compressed state resulting from a single compression operation, and wherein a contrast medium is present. Such breast preparation may be considered, for example, as an "initial activity" and may fall within block 410 of Fig. 4.

It can be understood from the above that a way has been found to determine a precise relationship (combination of the geometric parametrics of mapping and geometric transformation) between the 2D mammographic dual-energy image and the 3D tomosynthetic image; such a relation passes through the virtual 2D image. The virtual 2D image can be thought of as a "virtual" mammography that is not performed on a physical breast, but on a 3D reconstruction of the breast obtained by tomosynthesis.

Once this precise relationship has been determined, it is possible to switch precisely from dual energy mammography to tomosynthesis and vice versa; in particular, to display corresponding points and to know their spatial position, i.e., their coordinates.

This is useful when preparing for a biopsy to ensure that the biopsy needle withdraws a sample of tissue exactly at the point of the breast deemed as most relevant by the operator. Thus, the marking point referred to in step F of the method is conceptually related to the position of interest.

Suppose, for example, that a hypothetical lesion has been identified in the registered dual-energy mammography (this could derive from an operator observing it) and there is interest in identifying the lesion in the tomosynthesis and its spatial position; the operator may create, for example, a marking point on the 2D mammography image by selecting the point with a mouse (which will be highlighted); then, the operator can view one or more of the sections of the 3D tomosynthetic image and, thanks to the method according to the present invention, the marking point is automatically displayed in such sections; finally, the operator can evaluate if such marking point is suitable and signal it to the apparatus as a point to be reached with the biopsy.

From this example, it can be understood that the operator may be interested in operating (i.e., identifying marking points such as selecting points and/or moving points that have already been selected with the mouse) in the mammographic image and/or tomosynthetic section images. According to preferred embodiments of the present invention, both things are possible.

The aid method according to the present invention may provide displaying images on one or more screens; for example, it may be provided that a mammographic image (with possible marking elements) and an image of a tomosynthetic section (with possible marking elements) are displayed simultaneously on one or two screens or alternately on the same screen.

Typically, according to the present invention, steps A-F are performed in the order indicated above; indeed, it is very advantageous to perform mammography before tomosynthesis. However, changes of order are not excluded; for example, step B could be performed after step C.

It should be considered that the core of the present invention corresponds to steps D-F. In other words, according to certain embodiments, the aid method according to the present invention may start from a 2D mammographic dual energy image already acquired and from a 3D tomosynthetic image already acquired, under the assumption that they refer to the same breast in the same position, that is, without macro-movements of the breast as it always remains compressed.

According to the present invention, as is common practice, the 2D imaging procedure and the 3D imaging procedure are performed on a breast in a compressed state. Advantageously, according to the present invention, the 2D imaging procedure and the 3D imaging procedure (at least those used for steps E and F of the method) are performed on a breast in a compressed state resulting from a single compression operation.

Advantageously, according to the present invention, the 2D imaging procedure and the 3D imaging procedure (at least those used for steps E and F of the method) are performed on a breast in which a contrast medium is present. In particular, a dose of a contrast medium may be injected only once before the 2D imaging procedure because the timing of steps A, B and C is such that the contrast medium remains in circulation and both can benefit (even if steps A and B are repeated two or three times).

Preferably, according to the present invention, the 2D imaging procedure is carried out with the use of an X-ray grid, which is then removed before carrying out the 3D imaging procedure.

The method according to the present invention advantageously provides one or more repetitions of a performance of a 2D imaging procedure according to step A and a generation of a 2D composite image according to step B (i.e., repetitions of one or more dual energy mammographies) prior to performing steps A, B, C, D, E, F. Each of these repetitions typically derives from a specific request of an operator and is typically preceded by a preliminary operation of repositioning the breast with a subsequent preliminary operation of compressing the breast (which is distinct from the only compression operation mentioned above and on the basis of which steps D and E and F of the method are carried out).

It is useful to carry out a repetition if it is estimated that the biopsy procedure will not be positively reached. For example, an operator might look at a mammographic image and identify a hypothetical lesion that should be biopsied; then the operator, possibly assisted by a computer program, might estimate whether it is reachable, taking into account for example the estimated spatial position of the breast, the biopsy assembly and the position(s) of the access/withdrawal window(s) of the compressor; if this estimate is negative, i.e. the lesion is not reachable, the breast must be repositioned and the dual energy mammography must be repeated. According to the present document, as is common practice, the second energy mentioned in step A is greater than the first energy mentioned in step A; conceptually, it is irrelevant in a dual-energy mammography to first perform a "low energy" acquisition and then a "high energy" acquisition or vice versa; in practice, it may be preferable to start with the "low energy" acquisition (which also implies a lower total energy in the detector) to avoid "ghost" effects particularly evident in some types of X-ray detectors.

Preferably, according to the present invention, the third energy mentioned in step C is equal to or about equal to the second energy mentioned in step A, i.e., "high energy"; for example, the third energy may be lower than the second energy by, for example, 10-30%. In the case of different energies, the tomosynthesis imaging is performed at an energy such that there is a peak absorption of photons by the contrast medium, neglecting the optimisation of the visibility of the surrounding tissues (in contrast to conventional tomosynthesis). In this case, it may be appropriate to determine the transformation in step E of the method using, for example, the "high energy" image instead of the composite image.

In explaining step E of the method, reference was made to portions of the images to determine the transformation; this allows for instance to exclude irrelevant parts of the images (such as the "background") and/or to take in consideration only parts that are highly relevant for the biopsy. The portion of the image used in step E can be defined by the operator by, for example, drawing the outline of this portion on the screen. Alternatively, the portion of the image used in step E may be defined by a computer program and may correspond, for example, to an area of maximum reachability of the biopsy procedure, in particular enclosed by the profile of an access/withdrawal window of the compressor displayed on the screen.

In case of dual energy mammographies being repeated, it is preferable that the transformation referred to in step E of the method is determined on the basis of a 2D image deriving from the last performance of these two steps prior to step E, in particular the last composite 2D image generated or the last "high energy" 2D image acquired; in this way, there will be a greater relation between the 2D image and the 3D image.

Previously, when generally describing the method according to the present invention, reference was made to "at least one marking point". However, it is typical and advantageous of the present invention that in step F the operator and/or the computer program identifies (and highlights) a marking area surrounded by a closed marking line. In this case, the display can be focused on, for example, and be limited to the closed marking line.

According to the present invention, it is possible and advantageous that geometric mapping parametrics are used to display in one or more sections of the registered 3D image and/or in the 2D image points, lines or areas belonging to a volume calculated by a computer program; this may be useful, for example, to display the (simulated) passage cylinder of the biopsy needle.

Thus, in general according to the present invention, it is possible to display on a screen the registered 2D image and/or one or more sections of the 3D image as well as a marking point or a marking line or a marking area.

We will now consider more in detail image processing.

In step D, mapping may include:
- defining a 2D matrix of pixels in the predetermined acquisition plane,
- creating a plurality of projection axes in the 3D image, wherein these projection axes pass respectively through the pixels of the 2D matrix and through the predetermined emission point,
- associating a corresponding projection volume of the 3D image which surrounds the projection axis to each of the projection axes, wherein said projection volume is preferably cone- or pyramid-shaped,

- identifying for each projection volume the voxels of the 3D image that are internal (or at least partly internal) to the projection volume,
- for the pixels of the 2D matrix, choosing at least one function which defines an intensity value with respect to the intensity of the voxels of the corresponding projection volume.

Such a function may be "binary" (i.e., defined over two values) or "numeric" (i.e., defined over a discrete range of values) or a mixture of "numeric" and "binary" elements - since processing according to the present invention will be carried out by a computer, strictly speaking, one cannot speak of a "continuous range of values".

This function may be a combination (e.g., a linear combination) of maximum and/or minimum and/or mean and/or median.

In step E, the transformation may be of the geometric type; in particular, it may comprise: translation and/or rotation and/or uniform scaling and/or non-uniform scaling and/or shear deformation and/or perspective deformation (also called homographic transformation which is much preferred).

In step E, the transformation may be determined by a preliminary feature extraction from a portion of the reference 2D image (in particular the composite 2D image or the "high energy" 2D image) and from the corresponding portion of the virtual 2D image; consider for example the "high energy" image 1002 in Fig. 6 and the composite image 1003 in Fig. 7 and virtual image 1004 in Fig. 8.

The algorithm that allows the transformation to be achieved is complex and typically includes: a step of extracting the relevant characteristics or "features" from the two images, a step of creating the couplings for the features extracted from the two images and a step of identifying the geometric transformation that adapts one image to the other according to the couplings created.

Two aspects of the algorithm need to be considered with particular care during practical implementation: the choice of a number of "features" to be extracted from the two images and the constraints to be imposed on the "feature" couplings in order to filter out false matches. As the number of "features" increases, if the couplings are sufficiently reliable according to the constraints imposed, the accuracy with which the transformation is identified increases, but so does the time required to calculate the couplings.

According to the present invention, a "feature" extraction method of the ORB (= Oriented Fast and Rotated Brief) type is preferably employed in which a maximum number of "features" equal to about 1% of the total pixels composing the image under examination is preferably set, which is preferably segmented with respect to the background of the image (typically, therefore the background pixels are not considered); in order to reduce the calculation time, this limit could also be reduced for example to 0.1%. As already mentioned, extracting the features from an image under examination may only concern a portion of the image.

The ORB method basically consists of a combination of two algorithms called FAST (= Features from Accelerated Segment Test), which deals with "feature" extraction and BRIEF (= Binary Robust Independent Elementary Feature), which deals with the description of each individual feature. The FAST algorithm is based on the detection of contrast differences for each pixel in the image to which a "feature" will eventually be associated, and also allows features of different shapes and sizes to be identified thanks to a multi-scale analysis of the image (FAST can be iterated several times, each time sub-sampling the original image according to a geometric pyramid model). The BRIEF algorithm assigns a string of bits to each identified feature based on, for example, the shape and size of the feature, which constitutes the feature descriptor; the advantage of this bit-string representation is that feature descriptors can be processed more efficiently during the coupling process.

In order to determine the matches between the identified features, a matching method may advantageously be used that compares the descriptors (in pairs and in such a way that there is always a descriptor for each of the two images) for all the features identified in the two images and creates a coupling based, as a quantitative evaluation criterion, on the distance measure between the descriptors; preferably, the Hamming distance between two bit strings (which encode the descriptors) is calculated.

These methods are generally applicable to the analysis of any images and, therefore, the resulting matches do not take into account specific aspects related to, for example, the typical size of the lesion of interest and/or the typical size of the anatomical structures involved and/or their positioning. Therefore, according to the present invention, which is in the specific field of breast imaging, it is advantageous to provide for an additional filtering step which eliminates all obtained couplings not satisfying a number of spatial constraints. Among them, preferably an upper limit is set to the (Euclidean) distance for pairs of corresponding features (i.e. coupled according to the previous calculations) within the area of the breast under consideration in order to restrict the analysis only to consistent displacements with respect to the real movement of breast tissues during compression; this constraint can also preferably be obtained by estimating a weighted value (e.g. average displacement) from the analysis of the distribution of dislocations for pairs of corresponding features identified in the area of the breast under consideration. Feature pairs that do not fulfil the imposed constraint criteria are then discarded from the analysis, which leads to obtaining the transformation, ensuring a greater reliability.

Once the corresponding feature pairs of interest have been defined, in order to obtain the transformation used for registering, two vectors, usually called "source" and "target", are constructed, which contain the points with the two-dimensional coordinates of the features belonging respectively to the source image (e.g., the composite 2D image or a portion thereof - see image 1003 of Fig. 7) and the target image (e.g. the virtual 2D image or a portion thereof - see image 1004 of Fig. 8), ordered in such a way that the coupling matches are respected according to the vector index. Based on these two vectors, it is possible to derive a matrix that describes a transformation between the source image and the target image. This matrix can be used to transform the composite image and obtain a registered composite image. It should be noted that the transformation could also be applied to only a portion of the composite image, e.g., only to the portion used to calculate the transformation; in this case, a resulting image would be obtained that partly corresponds to the original composite image and partly corresponds to the registered composite image (a smoothing filter could be applied to the border).

Figures 5 to 8 relate to the same breast and are examples of images useful to understand the present invention. Fig. 5 and Fig. 6 show respectively a "low energy" and a "high energy" image captured during a 2D CESM imaging procedure; Fig. 7 shows a 2D "composite" image corresponding to a combination of the images in Fig. 5 and Fig. 6 - this image highlights a hypothetical lesion. Fig. 8 shows a "virtual" 2D image obtained by mapping a 3D tomosynthesis-type image onto the acquisition plane. In these images, the outline of the breast can be seen, as well as a darker frame due to a lower attenuation of the X-rays where they do not intercept the compressor at its access/withdrawal window for the biopsy needle (in case of access from a side, the compressor may not have any opening, but a simulated outline defining a zone of maximum reach for the needle may still be displayed). The accuracy purpose of the present invention is equal to or less than the accuracy imposed by the International Standard IEC 60601-2-45 regarding biopsy pointing, i.e., ±1 mm. This accuracy leads to registering the image in Fig. 7 with respect to the image in Fig. 8, although visually (given the scale of these two images) this would not seem necessary; it should be noted that displacements greater than 1 mm are unlikely for a breast under compression.

Fig. 9 shows the display of marking elements according to the present invention; the top image (Fig. 9A) is a registered "composite" 2D image and the images (Fig. 9B to Fig. 9F) below are related to (e.g. five) 2D sections of a 3D tomosynthetic image at different distances from the acquisition plane (as it can be understood from Fig. 3); the term "sections" is used even if they are slices of a small thickness (e.g. 0.5-1.0 mm).

In the image above (Fig. 9A), a radiologist has drawn a marking element; for example, a pentagon has been drawn around a hypothetical lesion. In all the images below (Fig. 9B to Fig. 9F), corresponding marking elements were displayed (automatically) by the method according to the present invention; it is noted that in some sections (Fig. 9B, Fig. 9C and Fig. 9D) within the marking element there is a figure, more or less clear, which corresponds to the hypothetical lesion, and that in other sections (Fig. 9E and Fig. 9F) there is nothing within the marking element corresponding to the hypothetical lesion, or rather suggesting a lesion. The radiologist (or a computer program) chooses the most suitable section for "biopsy pointing" based for example on the portion of the image within the marking element; for example, he or she might choose the section with the clearest figure (Fig. 9C). At this point, the radiologist can perform the "biopsy pointing" by typically identifying a point on the chosen section (Fig. 9C) as a "biopsy target"; consequently, a cross (centred on the point) is for example displayed in the section and subsequently (typically after an imperceptible time), a cross is for example displayed in the "composite" image by the method according to the present invention (note the cross both in Fig. 9A in Fig. 9C); alternatively or additionally, the outline of the section of a needle that will be used for sampling could be displayed. Different hatches and/or colours can help the operator distinguish between the markings he/she made directly from those produced automatically by the computer program. Once the radiologist is satisfied with the pointing he/she has carried out by the method according to the present invention, he/she can confirm the selection of the coordinates to the computer, which will then convert them into coordinates for the pointing system of the biopsy assembly so as to proceed with the biopsy examination.

The method according to the present invention is specifically adapted to be implemented in a mammography apparatus.

For example, the apparatus 1 in Fig. 1 includes means to implement this method. In particular, the processing and control unit and the human-machine interface unit are the essential means for implementing the method.

The core of the method for aiding an operator according to the present invention described above consists of steps D, E and F.

It can thus be defined as a computer-aided method which is carried out entirely by a computer electronic unit (such as a PC) and which comprises only steps D, E and F (and possibly also step B) and which operates on images stored in advance in a memory, e.g., an internal electronic or magnetic or optical memory or an external electronic or magnetic or optical memory. In this case, steps A and C (and possibly also step B) wherein there is (electromagnetic) interaction with a breast are carried out in advance (e.g., a few seconds before, or even less).

## Claims

1. Method for aiding an operator to determine a position of a region of interest in a breast, comprising the steps of:
A) (420, 430) performing a 2D imaging procedure of said breast comprising a first delivery (420) of X-rays from a predetermined emission point at a first energy to obtain a first image on a predetermined acquisition plane and a second delivery (430) of X-rays from said predetermined emission point at a second energy to obtain a second image on said predetermined acquisition plane, said predetermined emission point and said predetermined acquisition plane being predetermined geometric parameters of said 2D imaging procedure;
B) (440) generating by a processing and control unit a composite 2D image consisting of a 2D matrix of pixels corresponding to a combination of said first image and said second image;
C) (450) performing a tomosynthesis-type 3D imaging procedure of said breast by means of delivering X-rays from various emission points at a third energy to obtain a 3D image consisting of a 3D matrix;
D) (460) generating a virtual 2D image by means of a predetermined mapping of said 3D image on a virtual plane corresponding to said predetermined acquisition plane according to geometric mapping parameters corresponding to said predetermined geometric parameters;
E) (470, 480) determining (470) a two-dimensional transformation that links a portion of said first image or said second image or said composite 2D image and a corresponding portion of said virtual 2D image in such a way as to register said composite 2D image with respect to said virtual 2D image and to generate (480) a registered 2D image on the basis of said two-dimensional transformation; and
F) (490) using said geometric parameters of said mapping to display in a section of said 3D image at least one marking point previously identified by the operator and/or by a computer program in said registered 2D image or to display in said registered 2D image at least one marking point previously identified by the operator and/or by a computer program in a section of said 3D image;
wherein said 2D imaging procedure and said 3D imaging procedure are performed on a breast in a compressed state resulting from a single compression operation, and wherein a contrast medium is present;
wherein at least steps D, E and F are carried out by a computer electronic unit.

2. Method according to claim 1, wherein said 2D imaging procedure is performed using an X-ray grid.

3. Method according to claim 1 or 2, wherein said first energy is lower than said second energy, and wherein said second energy and said third energy are equal.

4. Method according to claim 1 or 2, wherein said first energy is lower than said second energy, and wherein said second energy and said third energy are different and in particular selected according to an energy of a photon absorption peak by said contrast medium.

5. Method according to claim 4, wherein said third energy is lower than said second energy by 10-30%.

6. Method according to any one of the preceding claims, comprising one or more repetitions of a 2D imaging procedure performance according to step A and a generation of a 2D composite image according to step B prior to performing said steps A, B, C, D, E, F, wherein, preferably, each repetition corresponds to a preliminary compression operation distinct from said single compression operation.

7. Method according to claim 6, wherein a repeat is made if a reachability estimation of a subsequent biopsy procedure is negative.

8. Method according to claim 6 or 7, wherein said step E is performed on the basis of the last first image obtained or last second image obtained or last composite 2D image generated.

9. Method according to any one of the preceding claims, wherein said image portion used in step E is defined by said operator.

10. Method according to any one of the preceding claims, wherein said image portion used in step E corresponds to an area of maximum reachability of a subsequent biopsy
procedure, in particular enclosed by a profile of an access/withdrawal window of a compressor plate of a mammography apparatus displayed on a screen.

11. Method according to any one of the preceding claims, wherein in said step F the operator and/or the computer program identifies a marking area surrounded by a closed marking line.

12. Method according to claim 11, wherein in said step F said geometric parameters of said mapping are used to display said closed marking line.

13. Method according to any one of the preceding claims, wherein said geometric parameters of said mapping are used to display points, lines or areas belonging to a volume calculated by a computer program in one or more sections of said 3D image and/or in said registered 2D image.

14. Method according to any one of the preceding claims, wherein said registered 2D image and/or one or more sections of said 3D image as well as a marking point or a marking line or a marking area are displayed on a screen.

15. Method according to any one of the preceding claims, wherein, in step D, said mapping comprises:
- defining a 2D matrix of pixels in said predetermined acquisition plane,
- creating a plurality of projection axes in said 3D image, wherein said projection axes pass respectively through the pixels of said 2D matrix and through said predetermined emission point,
- associating a corresponding projection volume of said 3D image which surrounds the projection axis to each of said projection axes, wherein said projection volume is preferably cone- or pyramid-shaped,
- identifying for each projection volume the voxels of said 3D image inside the projection volume,
- for the pixels of said 2D matrix, choosing at least one function which defines an intensity value with respect to the intensity of the voxels of the corresponding projection volume.

16. Method according to claim 15, wherein said function is a combination of maximum and/or minimum and/or mean and/or median.

17. Method according to any one of the preceding claims, wherein, in step E, said two-dimensional transformation is of the geometric type and comprises: translation and/or rotation and/or uniform scaling and/or non-uniform scaling and/or shear displacement and/or perspective displacement.

18. Method according to any one of the preceding claims, wherein, in step E, said two-dimensional transformation is determined by preliminarily extracting features from said portion of said first image or said second image or said composite 2D image and from said portion of said virtual 2D image.

19. Method according to any one of the preceding claims,
wherein said steps A and B and C or said steps A and C are performed in advance and create stored images, and
wherein said steps D and E and F or B and D and E and F are performed by operating on said stored images.

20. Mammography apparatus comprising means adapted to perform the method according to any one of the preceding claims.

## Patentansprüche

1. Verfahren zur Unterstützung eines Bedieners bei der Bestimmung der Position eines interessierenden Bereichs in einer Brust, umfassend die folgenden Schritte:
A) (420, 430) Durchführen eines 2D-Abbildungsverfahrens der Brust, umfassend eine erste Abgabe (420) von Röntgenstrahlen von einem vorbestimmten Emissionspunkt mit einer ersten Energie, um ein erstes Bild auf einer vorbestimmten Aufnahmeebene zu erhalten, und eine zweite Abgabe (430) von Röntgenstrahlen von dem vorbestimmten Emissionspunkt mit einer zweiten Energie, um ein zweites Bild auf der vorbestimmten Aufnahmeebene zu erhalten, wobei der vorbestimmte Emissionspunkt und die vorbestimmte Aufnahmeebene vorbestimmte geometrische Parameter des 2D-Abbildungsverfahrens sind;
B) (440) Erzeugen eines zusammengesetzten 2D-Bildes durch eine Verarbeitungs- und Steuereinheit, das aus einer 2D-Matrix von Pixeln besteht, die einer Kombination aus dem ersten Bild und dem zweiten Bild entsprechen;
C) (450) Durchführen eines 3D-Abbildungsverfahrens vom Typ Tomosynthese der Brust durch Abgabe von Röntgenstrahlen von verschiedenen Emissionspunkten mit einer dritten Energie, um ein 3D-Bild zu erhalten, das aus einer 3D-Matrix besteht;
D) (460) Erzeugen eines virtuellen 2D-Bildes mittels einer vorbestimmten Abbildung des 3D-Bildes auf eine virtuelle Ebene, die der vorbestimmten Aufnahmeebene entspricht, gemäß geometrischen Abbildungsparametern, die den vorbestimmten geometrischen Parametern entsprechen;
E) (470, 480) Bestimmen (470) einer zweidimensionalen Transformation, die einen Abschnitt des ersten Bildes oder des zweiten Bildes oder des zusammengesetzten 2D-Bildes und einen entsprechenden Abschnitt des virtuellen 2D-Bildes so verbindet, dass das zusammengesetzte 2D-Bild in Bezug auf das virtuelle 2D-Bild registriert wird und ein registriertes 2D-Bild auf der Grundlage der zweidimensionalen Transformation erzeugt wird (480); und
F) (490) Verwenden der geometrischen Parameter der Abbildung, um in einem Ausschnitt des 3D-Bildes mindestens einen Markierungspunkt anzuzeigen, der zuvor von dem Bediener und/oder von einem Computerprogramm in dem registrierten 2D-Bild identifiziert wurde, oder um in dem registrierten 2D-Bild mindestens einen Markierungspunkt anzuzeigen, der zuvor von dem Bediener und/oder von einem Computerprogramm in einem Ausschnitt des 3D-Bildes identifiziert wurde;
wobei das 2D-Abbildungsverfahrens und das 3D-Abbildungsverfahren an einer Brust in einem komprimierten Zustand durchgeführt werden, der sich aus einem einzigen Kompressionsvorgang ergibt, und
wobei ein Kontrastmittel vorhanden ist;
wobei mindestens die Schritte D, E und F von einer elektronischen Rechnereinheit durchgeführt werden.

2. Verfahren nach Anspruch 1, wobei das 2D-Abbildungsverfahren unter Verwendung eines Röntgenstrahlgitters durchgeführt wird.

3. Verfahren nach Anspruch 1 oder 2, wobei die erste Energie niedriger ist als die zweite Energie und wobei die zweite Energie und die dritte Energie gleich sind.

4. Verfahren nach Anspruch 1 oder 2, wobei die erste Energie niedriger als die zweite Energie ist, und wobei die zweite Energie und die dritte Energie unterschiedlich sind und insbesondere entsprechend einer Energie eines Photonenabsorptionspeaks durch das Kontrastmittel ausgewählt werden.

5. Verfahren nach Anspruch 4, wobei die dritte Energie um 10 - 30% niedriger ist als die zweite Energie.

6. Verfahren nach einem der vorstehenden Ansprüche, umfassend eine oder mehrere Wiederholungen einer 2D-Abbildungsverfahrensausführung gemäß Schritt A und eine Erzeugung eines zusammengesetzten 2D-Bildes gemäß Schritt B vor der Durchführung der Schritte A, B, C, D, E, F umfasst, wobei vorzugsweise jede Wiederholung einem vorläufigen Kompressionsvorgang entspricht, der sich von dem einzelnen Kompressionsvorgang unterscheidet.

7. Verfahren nach Anspruch 6, wobei eine Wiederholung durchgeführt wird, wenn eine Erreichbarkeitsabschätzung eines nachfolgenden Biopsieverfahrens negativ ist.

8. Verfahren nach Anspruch 6 oder 7, wobei der Schritt E auf der Grundlage des zuletzt erhaltenen ersten Bildes oder des zuletzt erhaltenen zweiten Bildes oder des zuletzt erzeugten zusammengesetzten 2D-Bildes durchgeführt wird.

9. Verfahren nach einem der vorstehenden Ansprüche, wobei der in Schritt E verwendete Bildabschnitt von dem Bediener definiert wird.

10. Verfahren nach einem der vorstehenden Ansprüche, wobei der in Schritt E verwendete Bildabschnitt einem Bereich maximaler Erreichbarkeit eines nachfolgenden Biopsieverfahrens entspricht, der insbesondere von einem Profil eines auf einem Bildschirm dargestellten Zugangs-/Entnahmefensters einer Kompressorplatte eines Mammographiegerätes umschlossen wird.

11. Verfahren nach einem der vorstehenden Ansprüche, wobei in dem Schritt F der Bediener und/oder das Computerprogramm einen Markierungsbereich identifiziert, der von einer geschlossenen Markierungslinie umgeben ist.

12. Verfahren nach Anspruch 11, wobei in dem Schritt F die geometrischen Parameter der Abbildung verwendet werden, um die geschlossene Markierungslinie anzuzeigen.

13. Verfahren nach einem der vorstehenden Ansprüche, wobei die geometrischen Parameter der Abbildung verwendet werden, um Punkte, Linien oder Flächen, die zu einem von einem Computerprogramm berechneten Volumen gehören, in einem oder mehreren Ausschnitten des 3D-Bildes und/oder in dem registrierten 2D-Bild anzuzeigen.

14. Verfahren nach einem der vorstehenden Ansprüche, wobei das registrierte 2D-Bild und/oder ein oder mehrere Ausschnitte des 3D-Bildes sowie ein Markierungspunkt oder eine Markierungslinie oder ein Markierungsbereich auf einem Bildschirm angezeigt werden.

15. Verfahren nach einem der vorstehenden Ansprüche, wobei in Schritt D die Abbildung umfasst:
- Definieren einer 2D-Matrix von Pixeln in der vorbestimmten Aufnahmeebene,
- Erzeugen einer Vielzahl von Projektionsachsen in dem 3D-Bild, wobei die Projektionsachsen jeweils durch die Pixel der 2D-Matrix und durch den vorbestimmten Emissionspunkt verlaufen,
- Zuordnen eines entsprechenden Projektionsvolumens des 3D-Bildes, das die Projektionsachse umgibt, zu jeder der Projektionsachsen, wobei das Projektionsvolumen vorzugsweise kegel- oder pyramidenförmig ist,
- Identifizieren, für jedes Projektionsvolumen, der Voxel des 3D-Bildes innerhalb des Projektionsvolumens,
- Auswählen, für die Pixel der genannten 2D-Matrix, von mindestens einer Funktion, die einen Intensitätswert in Bezug auf die Intensität der Voxel des entsprechenden Projektionsvolumens definiert.

16. Verfahren nach Anspruch 15, wobei die Funktion eine Kombination aus Maximum und/oder Minimum und/oder Mittelwert und/oder Median ist.

17. Verfahren nach einem der vorstehenden Ansprüche, wobei in Schritt E die zweidimensionale Transformation vom geometrischen Typ ist und Folgendes umfasst: Translation und/oder Rotation und/oder gleichmäßige Skalierung und/oder ungleichmäßige Skalierung und/oder Schubverschiebung und/oder perspektivische Verschiebung.

18. Verfahren nach einem der vorstehenden Ansprüche, wobei in Schritt E die zweidimensionale Transformation durch vorheriges Extrahieren von Merkmalen aus dem Abschnitt des ersten Bildes oder des zweiten Bildes oder des zusammengesetzten 2D-Bildes und aus dem Abschnitt des virtuellen 2D-Bildes bestimmt wird.

19. Verfahren nach einem der vorstehenden Ansprüche,
wobei die Schritte A und B und C oder die Schritte A und C im Voraus durchgeführt werden und gespeicherte Bilder erzeugen, und
die Schritte D und E und F oder B und D und E und F durch Bearbeitung der gespeicherten Bilder durchgeführt werden.

20. Mammographiegerät mit Mitteln zur Durchführung des Verfahrens nach einem der vorstehenden Ansprüche

## Revendications

1. Procédé pour aider un opérateur à déterminer une position d'une région d'intérêt dans un sein, comprenant les étapes suivantes :
A) (420, 430) la réalisation d'une procédure d'imagerie 2D dudit sein comprenant une première émission (420) de rayons X à partir d'un point d'émission prédéterminé à une première énergie pour obtenir une première image sur un plan d'acquisition prédéterminé et une deuxième émission (430) de rayons X à partir dudit point d'émission prédéterminé à une deuxième énergie pour obtenir une deuxième image sur ledit plan d'acquisition prédéterminé, ledit point d'émission prédéterminé et ledit plan d'acquisition prédéterminé étant des paramètres géométriques prédéterminés de ladite procédure d'imagerie 2D ;
B) (440) la génération, par une unité de traitement et de commande, d'une image 2D composite constituée d'une matrice 2D de pixels correspondant à une combinaison de ladite première image et de ladite deuxième image ;
C) (450) la réalisation d'une procédure d'imagerie 3D de type tomosynthèse dudit sein au moyen de l'émission de rayons X à partir de différents points d'émission à une troisième énergie pour obtenir une image 3D constituée d'une matrice 3D ;
D) (460) la génération d'une image 2D virtuelle au moyen d'une cartographie prédéterminée de ladite image 3D sur un plan virtuel correspondant audit plan d'acquisition prédéterminé selon des paramètres de mappage géométrique correspondant auxdits paramètres géométriques prédéterminés ;
E) (470, 480) la détermination (470) d'une transformation bidimensionnelle qui relie une partie de ladite première image ou de ladite deuxième image ou de ladite image 2D composite et une partie correspondante de ladite image 2D virtuelle de manière à enregistrer ladite image 2D composite par rapport à ladite image 2D virtuelle et à générer (480) une image 2D enregistrée sur la base de ladite transformation bidimensionnelle ; et
F) (490) l'utilisation desdits paramètres géométriques de ladite cartographie pour afficher dans une section de ladite image 3D au moins un point de marquage préalablement identifié par l'opérateur et/ou par un programme informatique dans ladite image 2D enregistrée ou pour afficher dans ladite image 2D enregistrée au moins un point de marquage préalablement identifié par l'opérateur et/ou par un programme informatique dans une section de ladite image 3D;
dans lequel ladite procédure d'imagerie 2D et ladite procédure d'imagerie 3D sont réalisées sur un sein dans un état comprimé résultant d'une opération de compression unique, et
dans lequel un agent de contraste est présent ;
dans lequel au moins les étapes D, E et F sont réalisées par une unité électronique informatique.

2. Procédé selon la revendication 1, dans lequel ladite procédure d'imagerie 2D est réalisée à l'aide d'une grille à rayons X.

3. Procédé selon la revendication 1 ou 2, dans lequel ladite première énergie est inférieure à ladite deuxième énergie, et dans lequel ladite deuxième énergie et ladite troisième énergie sont égales.

4. Procédé selon la revendication 1 ou 2, dans lequel ladite première énergie est inférieure à ladite deuxième énergie, et dans lequel ladite deuxième énergie et ladite troisième énergie sont différentes et notamment sélectionnées en fonction d'une énergie d'un pic d'absorption de photons par ledit agent de contraste.

5. Procédé selon la revendication 4, dans lequel ladite troisième énergie est inférieure à ladite deuxième énergie de 10 à 30 %.

6. Procédé selon l'une quelconque des revendications précédentes, comprenant une ou plusieurs répétitions d'une réalisation de procédure d'imagerie 2D selon l'étape A et d'une génération d'une image composite 2D selon l'étape B avant de réaliser lesdites étapes A, B, C, D, E, F, dans lequel, de préférence, chaque répétition correspond à une opération de compression préliminaire distincte de ladite opération de compression unique.

7. Procédé selon la revendication 6, dans lequel une répétition est effectuée si une estimation d'accessibilité d'une procédure de biopsie ultérieure est négative.

8. Procédé selon la revendication 6 ou 7, dans lequel ladite étape E est réalisée sur la base de la dernière première image obtenue ou de la dernière deuxième image obtenue ou de la dernière image composite 2D générée.

9. Procédé selon l'une quelconque des revendications précédentes, dans lequel ladite partie d'image utilisée dans l'étape E est définie par ledit opérateur.

10. Procédé selon l'une quelconque des revendications précédentes, dans lequel ladite partie d'image utilisée dans l'étape E correspond à une zone d'accessibilité maximale d'une procédure de biopsie ultérieure, notamment délimitée par un profil d'une fenêtre d'accès/retrait d'une plaque de compression d'un appareil de mammographie affichée sur un écran.

11. Procédé selon l'une quelconque des revendications précédentes, dans lequel, dans ladite étape F, l'opérateur et/ou le programme informatique identifie une zone de marquage entourée d'une ligne de marquage fermée.

12. Procédé selon la revendication 11, dans lequel, dans ladite étape F, lesdits paramètres géométriques de ladite cartographie sont utilisés pour afficher ladite ligne de marquage fermée.

13. Procédé selon l'une quelconque des revendications précédentes, dans lequel lesdits paramètres géométriques de ladite cartographie sont utilisés pour afficher des points, des lignes ou des zones appartenant à un volume calculé par un programme informatique dans une ou plusieurs sections de ladite image 3D et/ou dans ladite image 2D enregistrée.

14. Procédé selon l'une quelconque des revendications précédentes, dans lequel ladite image 2D enregistrée et/ou une ou plusieurs sections de ladite image 3D ainsi qu'un point de marquage ou une ligne de marquage ou une zone de marquage sont affichés sur un écran.

15. Procédé selon l'une quelconque des revendications précédentes, dans lequel, dans l'étape D, ladite cartographie comprend :
- la définition d'une matrice 2D de pixels dans ledit plan d'acquisition prédéterminé,
- la création d'une pluralité d'axes de projection dans ladite image 3D, dans lequel lesdits axes de projection passent respectivement par les pixels de ladite matrice 2D et par ledit point d'émission prédéterminé,
- l'association d'un volume de projection correspondant de ladite image 3D qui entoure l'axe de projection à chacun desdits axes de projection, dans lequel ledit volume de projection est de préférence en forme de cône ou de pyramide,
- l'identification, pour chaque volume de projection, des voxels de ladite image 3D à l'intérieur du volume de projection,
- pour les pixels de ladite matrice 2D, le choix d'au moins une fonction qui définit une valeur d'intensité par rapport à l'intensité des voxels du volume de projection correspondant.

16. Procédé selon la revendication 15, dans lequel ladite fonction est une combinaison de maximum et/ou de minimum et/ou de moyenne et/ou de médiane.

17. Procédé selon l'une quelconque des revendications précédentes, dans lequel, dans l'étape E, ladite transformation bidimensionnelle est de type géométrique et comprend : une translation et/ou une rotation et/ou une mise à l'échelle uniforme et/ou une mise à l'échelle non uniforme et/ou un déplacement en cisaillement et/ou un déplacement en perspective.

18. Procédé selon l'une quelconque des revendications précédentes, dans lequel, dans l'étape E, ladite transformation bidimensionnelle est déterminée en extrayant préalablement des caractéristiques de ladite partie de ladite première image ou de ladite deuxième image ou de ladite image 2D composite et de ladite partie de ladite image 2D virtuelle.

19. Procédé selon l'une quelconque des revendications précédentes,
dans lequel lesdites étapes A et B et C ou lesdites étapes A et C sont réalisées à l'avance et créent des images stockées, et
dans lequel lesdites étapes D et E et F ou B et D et E et F sont réalisées en opérant sur lesdites images stockées.

20. Appareil de mammographie comprenant des moyens adaptés réaliser le procédé selon l'une quelconque des revendications précédentes.
